(19) Europäisches Patentamt
European Patent Office
Office européen des brevets

(11) Veröffentlichungsnummer: **0 396 987 B1**

(12) **EUROPÄISCHE PATENTSCHRIFT**

(45) Veröffentlichungstag der Patentschrift: **15.06.94**

(51) Int. Cl.$^5$: **C07C 47/55**, C07C 45/63

(21) Anmeldenummer: **90108150.5**

(22) Anmeldetag: **28.04.90**

# (54) Kernfluorierte Trifluormethylbenzaldehyde.

(30) Priorität: **12.05.89 DE 3915495**

(43) Veröffentlichungstag der Anmeldung:
**14.11.90 Patentblatt 90/46**

(45) Bekanntmachung des Hinweises auf die Patenterteilung:
**15.06.94 Patentblatt 94/24**

(84) Benannte Vertragsstaaten:
**BE CH DE FR GB IT LI**

(56) Entgegenhaltungen:
**EP-A- 0 125 803**
**EP-A- 0 265 854**
**EP-A- 0 289 942**
**EP-A- 0 347 692**

**J. Hendrickson, D. Cram, G. Hammond: "Organic Chemistry 3rd edn." 1970, McGraw-Hill, Düsseldorf, S. 653**

**HOUBEN-WEYL, METHODEN DER ORGANISCHEN CHEMIE, V/3, S. 162, 163, Abschnitt bb), 1962**

**ORGANISCHE CHEMIE, Walter Gruyter-Verlag, Berlin-New York, 1980, S. 570, 576, N. L. Allinger et al**

(73) Patentinhaber: **BAYER AG**

**D-51368 Leverkusen(DE)**

(72) Erfinder: **Marhold, Albrecht, Dr.**
**Carl-Duisberg-Strasse 329**
**D-5090 Leverkusen 1(DE)**
Erfinder: **Braden, Rudolf, Dr.**
**Nothauser Feld 1**
**D-5068 Odenthal(DE)**

**EP 0 396 987 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung kernfluorierter Trifluormethylbenzaldehyde, die gegebenenfalls zusätzlich kernchloriert sein können, und neue kernfluorierte, gegebenenfalls auch kernchlorierte Trifluormethylbenzaldehyde.

Es ist bekannt, kernchlorierte, keine $CF_3$-Gruppen enthaltende Benzaldehyde mit Kaliumfluorid in die entsprechenden kernfluorierten Benzaldehyde überzuführen (siehe Chemistry Letters 1988, S. 1355-1358). Dort ist aus Tabelle 1 ersichtlich, daß nur in Gegenwart von Tetraphenylphosphoniumsalzen und bestimmten Ethern (z.B. 18-Krone-6) Ausbeuten von ca. 70 % realisiert werden können, wogegen Tetraphenylphosphoniumsalze alleine, Ether alleine und die Kombination eines Tetraalkylphosphoniumsalzes mit einem Ether sehr schlechte Ausbeuten (nur zwischen 43 und 5 %) ergeben. Bei einem derart sensiblen System kann nicht vorhergesehen werden, wie sich eine zusätzliche $CF_3$-Gruppe, die die Elektronenverteilung am aromatischen Kern stark ändert, auf die Möglichkeiten von Chlor-Fluor-Austauschreaktionen auswirkt.

Aus der EP-A 125 803 (siehe Beispiel T) ist es bekannt 2-Chlor-3-trifluormethyl-5-fluor-benzaldehyd und 2-Trifluormethyl-3-chlor-6-fluor-benzaldehyd herzustellen, indem man in 1-Chlor-2-trifluormethyl-4-fluor-benzol unter Verwendung von Butyllithium eine CHO-Gruppe einführt. Die Handhabung von Butyllithium erfordert besonderen technischen Aufwand. Außerdem sind die erzielbaren Ausbeuten gering. Für eine Anwendung im technischen Maßstab ist diese Arbeitsweise deshalb wenig geeignet.

Ein zur EP-A 125 803 analoges Verfahren zur Herstellung von 2-Trifluormethyl-3,6-difluor-benzaldehyd, 2,5-Difluor-3-trifluormethyl-benzaldehyd und 2-Trifluormethyl-3,5-dichlor-6-fluor-benzaldehyd ist aus der EP-A 174 131 bekannt (siehe Beispiele C und H).

Aus EP-A 265 854 geht hervor, daß man in Chlorbenzaldehyden Chloratome in 2- und/oder 4-Stellung gegen Fluoratome austauschen kann. Die 3- und 5-Positionen am Benzolkern können dabei gegebenenfalls mit einem die Elektronendichte herabsetzenden Rest, wie Nitro-, Cyano- oder weiteren Formylgruppen, substituiert sein. Welche Auswirkungen $CF_3$-Substituenten auf den Chlor/Fluor-Austausch haben ist nicht beschrieben.

In Houben-Weyl, Methoden der organischen Chemie, Band VI/3, Seiten 162 und 163, Abschnitt *β.β*-(1962) ist der Austausch von Chlor gegen Fluor in 1,3-Dinitro- und 1-Trifluormethyl-3-nitro-Aromaten beschrieben. Voraussagen über das Verhalten von Trifluormethyl- und Formylgruppenenthaltenden Aromaten bei solchen Austauschreaktionen können auch hier nicht gemacht werden.

N.L Allinger et al, Organische Chemie, Walter Gruyter-Verlag, Berlin-New York (1980), Seiten 570 und 576 geben an, daß eine $CF_3$-Gruppe hinsichtlich ihres Einflusses auf aromatische Systeme mit $NH_3^+$- und COZ-Gruppen verglichen werden könne. Für die $NH_3^+$-Gruppe ist eine stark desaktivierende und stark meta-dirigierende Wirkung beschrieben, für die CHO-Gruppe eine mittelstark desaktivierende und schwach meta-dirigierende Wirkung. Man kann auch hier nicht erkennen, wie sich eine $CF_3$-Gruppe auf den Chlor/Fluor-Austausch auswirkt.

Es wurde nun ein Verfahren zur Herstellung von kernfluorierten Trifluormethylbenzaldehyden der Formel (I) gefunden,

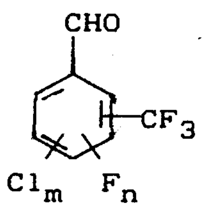

(I).

in der

n für 1, 2 oder 3 und

m für 0, 1 oder 2 steht, wobei

n + m maximal 3 beträgt,

das dadurch gekennzeichnet ist, daß man einen kernchlorierten Trifluormethylbenzaldehyd der Formel (II),

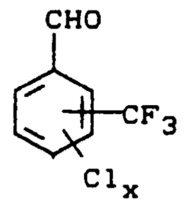

(II),

in der

x für 1, 2 oder 3 steht,

mit Kaliumfluorid bei erhöhter Temperatur in Gegenwart eines Lösungsmittels und in Abwesenheit von Wasser umsetzt.

In das erfindungsgemäße Verfahren einsetzbare kernchlorierte Trifluormethylbenzaldehyde der Formel (II), z.B. 4-Chlor-3-trifluormethyl-, 2,3-Dichlor-4-trifluormethyl-, 4,5-Dichlor-2-trifluormethyl-, 2,6-Dichlor-3-trifluormethyl-, 2,4-Dichlor-5-trifluormethyl-, 2,6-Dichlor-4-trifluormethyl-, 2,5-Dichlor-4-trifluormethyl-, 2-Chlor-5-trifluormethyl-, 4-Chlor-2-trifluormethyl-, 2-Chlor-3-trifluormethyl-, 2-Chlor-4-trifluormethyl-, 2,4,6-Trichlor-3-trifluormethyl-, 2,3,6-Trichlor-4-trifluormethyl-, 2,3,5-Trichlor-4-trifluormethyl-, 2,3,4-Trichlor-6-trifluormethyl- und 2,3,4-Trichlor-5-trifluormethyl-benzaldehyd, sind größtenteils bekannte Verbindungen, die beispielsweise gemäß Houben-Weyl, Methoden der org. Chemie, Band E 3, Seite 350 ff (1983) erhältlich sind. Soweit es sich bei Formel (II) um neue Verbindungen handelt, sind diese auf analoge Weise wie die bekannten Verbindungen zugänglich.

Das Kaliumfluorid kann beispielsweise in einer Menge von 1 bis 2 Mol pro Mol gegen Fluor auszutauschendes Chlor eingesetzt werden. Vorzugsweise ist diese Menge 1,4 bis 1,6 Mol pro Mol auszutauschendes Chlor.

Das erfindungsgemäße Verfahren kann beispielsweise bei Temperaturen im Bereich 160 bis 210 °C durchgeführt werden. Bevorzugt arbeitet man bei 180 bis 200 °C. Im allgemeinen arbeitet man bei Normaldruck. Ein Arbeiten unter Druck oder in einem geschlossenen Gefäß kann erforderlich werden, falls die eingesetzte Verbindung der Formel (II), die Reaktionsprodukte der Formel (I) und/oder das eingesetzte Lösungsmittel bei der gewünschten Reaktionstemperatur flüchtig sind.

Als Lösungsmittel kommen insbesondere polare, aprotische Lösungsmittel in Frage, beispielsweise Tetramethylensulfon, N-Methylpyrrolidon, Dimethylacetamid, Dimethylformamid, N-Methylcaprolactam und Dimethylsulfoxid.

Eine ausreichende Abwesenheit von Wasser kann z.B. erreicht werden, indem man wasserfreie Lösungsmittel und wasserfreie Einsatzprodukte der Formel (II) verwendet. Kaliumfluorid enthält häufig kleine Mengen Wasser. Dieses kann man beispielsweise entfernen, indem man zunächst nur Kaliumfluorid und Lösungsmittel in das Reaktionsgefäß einbringt und das Wasser zusammen mit etwas Lösungsmittel abdestilliert. Vorhandenes Wasser kann man auch entfernen, indem man eine aromatische Verbindung, z.B. Toluol oder Xylol, zusetzt und das Wasser als Azeotrop mit der aromatischen Verbindung abdestilliert.

Das erfindungsgemäße Verfahren kann in Anwesenheit oder Abwesenheit von Katalysatoren durchgeführt werden. Als Katalysatoren kommen beispielsweise quartäre Salze, wie Ammonium- oder Phosphoniumsalze, gegebenenfalls mit Zusätzen von Salzen von Übergangsmetallen und/oder Kronenether, in Frage. Katalysatoren können beispielsweise in Mengen von 0,2 bis 5 Gew.-%, eingesetzt werden, bezogen auf den eingesetzten kernchlorierten Trifluormethylbenzaldehyd der Formel (II). Weiterhin können Caesiumsalze, bevorzugt Halogenide, als zusätzliche Katalysatoren verwendet werden. Die Mengen lagen hierbei aus ökonomischen Gründen beispielsweise bei 0,2-10 %, können jedoch auch höher liegen.

Aus dem nach der Umsetzung vorliegenden Reaktionsgemisch kann man das oder die hergestellten Produkte z.B. durch Destillation abtrennen und reinigen. Man kann dabei auch Vakuum- oder Wasserdampf-Destillation anwenden.

Wenn der eingesetzte kernchlorierte Trifluormethylbenzolaldehyd mehrere Chloratome enthält (Formel (II), x = 2 oder 3), so können ein Chloratom oder mehrere oder alle

Chloratome gegen Fluor ausgetauscht werden. Ein selektiver Chlor-/Fluor-Austausch ist insbesondere bei solchen Chloratomen möglich, die in o- oder p-Position zur CHO-Gruppe vorliegen. Chloratome, die in m-Position zur CHO-Gruppe vorliegen, bleiben dann bei entsprechender Dosierung von Kaliumfluorid und bei Anwendung nicht allzu scharfer Reaktionsbedingungen unausgetauscht am Benzolkern. So kann beispielsweise aus 4,5-Dichlor-2-trifluormethyl-benzaldehyd in guten Ausbeuten 4-Fluor-5-chlor-2-trifluormethylbenzaldehyd hergestellt werden. Bei entsprechender Dosierung von Kaliumfluorid und bei Anwendung

relativ scharfer Reaktionsbedingungen werden im allgemeinen sämtliche am benzolischen Kern vorhandenen Chloratome gegen Fluor ausgetauscht.

Es wurden weiterhin neue kernfluorierte Trifluormethylbenzaldehyde der Formel (Ia) gefunden

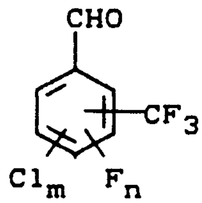

(Ia),

in der

n für 1, 2 oder 3 und

m für 0, 1 oder 2 steht, wobei

n + m 2 oder 3 beträgt und

wobei die gemäß EP-A 125 803 und EP-A 174 131 hergestellten kernfluorierten Trifluormethylbenzaldehyde ausgenommen sind.

Bevorzugte Verbindungen der Formel (Ia) sind:

2-Trifluormethyl-4,6-difluor-benzaldehyd,
2-Trifluormethyl-4-fluor-6-chlor-benzaldehyd,
2-Trifluormethyl-4-fluor-5-chlor-benzaldehyd,
2-Trifluormethyl-4,5-difluor-benzaldehyd,
3-Trifluormethyl-4,5-difluor-benzaldehyd,
3-Trifluormethyl-4-fluor-5-chlor-benzaldehyd,
2,4-Difluor-3-trifluormethyl-benzaldehyd,
3-Trifluormethyl-4,6-difluor-benzaldehyd,
2-Fluor-4-trifluormethyl-5-chlor-benzaldehyd,
2-Fluor-3-chlor-4-trifluormethyl-benzaldehyd,
2,4-Difluor-4-trifluormethyl-benzaldehyd,
2,6-Difluor-4-trifluormethyl-benzaldehyd,
2-Chlor-4-trifluormethyl-6-fluor-benzaldehyd,
3-Chlor-4-trifluormethyl-6-fluor-benzaldehyd,
2,6-Difluor-4-trifluormethyl-5-chlor-benzaldehyd,
2,5,6-Trifluor-3-trifluormethyl-benzaldehyd,
2,6-Difluor-3-trifluormethyl-5-chlor-benzaldehyd,
2,4,6-Trifluor-3-trifluormethyl-benzaldehyd,
2-Trifluormethyl-3-chlor-4,6-difluor-benzaldehyd und
2-Trifluormethyl-3,4,6-trifluor-benzaldehyd.

Die Verbindungen der Formel (Ia) sind erhältlich wie weiter oben beschrieben.

Kernfluorierte Trifluormethylbenzaldehyde der Formel (I) sind wichtige Zwischenprodukte zur Herstellung von pharmazeutisch wirksamen Dihydropyridinen. Solche Dihydropyridine können z.B. gemäß der EP-A 125 803 und EP-A 174 131 oder in analoger Weise dazu hergestellt werden. Die erfindungsgemäß zugänglichen neuen kernfluorierten Trifluormethylbenzaldehyde der Formel (Ia) erweitern den Umfang der so zugänglichen Dihydropyridine.

Bei dem eingangs geschilderten Stand der Technik ist es ausgesprochen überraschend, daß es erfindungsgemäß auf einfache und im technischen Maßstab gut realisierbare Weise gelingt, bekannte und neue kernfluorierte Trifluormethylbenzaldehyde herzustellen.

## Beispiele

### Beispiel 1

50 g Kaliumfluorid wurden in 120 ml Tetramethylensulfon suspendiert und zur vollständigen Trocknung bei 15 mbar andestilliert. Anschließend wurden 60 g 4-Chlor-3-trifluormethyl-benzaldehydzugegeben und für 7 Stunden unter Feuchtigkeitsausschluß auf 190 °C erhitzt. Die Temperatur fiel im Verlauf der Reaktion auf 180 °C ab, gleichzeitig trat Sieden am Rückfluß ein. Danach wurde gaschromatographisch ein Umsatz von über 96 % angezeigt. Das so erhaltene Reaktionsgemisch wurde einer Wasserdampfdestillation unterworfen

und nach Abtrennen des Produktes aus dem Destillat redestilliert. Es wurden 45 g 4-Fluor-3-trifluormethyl-benzaldehyd mit einem Siedepunkt von 70 bis 72 °C bei 12 mbar erhalten.

Beispiel 2

Analog Beispiel 1 wurden 92 g Kaliumfluorid in 290 ml Tetramethylensulfon mit 116 g 2,3-Dichlor-4-trifluormethyl-benzaldehyd in Gegenwart von 5 g Tributylbenzylphosphoniumchlorid für 7 Stunden erhitzt. Danach wurden 16 g 2,3-Difluor-4-trifluormethyl-benzaldehyd (Siedepunkt 62 bis 64 °C bei 20 mbar, Brechungsindex $n_D^{20}$ : 1,4535) und 66 g 2-Fluor-3-chlor-4-trifluormethyl-benzaldehyd (Siedepunkt 88 bis 90 °C bei 20 mbar, Brechungsindex $n_D^{20}$ : 1,4875) erhalten.

Beispiel 3

Eine Mischung aus 106 g trockenem Kaliumfluorid in 190 ml Tetramethylensulfon (bei 15 mbar zur Trocknung andestilliert), 6,6 g Tributylbenzylphosphoniumchlorid, 2,6 g Eisen-III-chlorid und 132 g 4,5-Dichlor-2-trifluormethyl-benzaldyd wurde für 7 Stunden bei 190 °C umgesetzt. Nach dem Abkühlen des Ansatzes wurde das Reaktionsgemisch bei 15 mbar grob destilliert. Die Feindestillation des Destillates ergab:
26 g 4,5-Difluor-2-trifluormethyl-benzaldehyd mit einem Siedepunkt von 42 bis 45 °C bei 12 bar und einem Brechungsindex $n_D^{20}$ von 1,4375 und
57 g 5-Chlor-4-fluor-2-trifluormethyl-benzaldehyd mit einem Siedepunkt von 69 bis 70 °C bei 12 mbar und einem Brechungsindex $n_D^{20}$ von 1,4745.

Beispiel 4

Beispiel 3 wurde wiederholt, jedoch wurde 5 Stunden auf eine Temperatur von 200 °C erhitzt. Es wurden 42 g der Difluorverbindung und 41 g der Fluor-chlor-Verbindung erhalten.

Beispiel 5

Analog Beispiel 1 wurden 40 g Kaliumfluorid in 150 ml Tetramethylensulfon vorgelegt und im Vakuum andestilliert, dann zusammen mit 3 g Tributyl-benzyl-phosphoniumchlorid, 1 g Eisen-III-chlorid und 50 g 2,5-Dichlor-3-trifluormethyl- benzaldehyd für 7 Stunden auf 185 bis 190 °C erhitzt. Die Destillation im Vakuum ergab 39 g 2,6-Difluor-3-trifluormethyl-benzaldehyd mit einem Siedepunkt von 49 bis 51 °C bei 10 mbar und einem Brechungsindex von $n_D^{20}$ von 1,4492.

Herstellung von einigen Ausgangsprodukten der Formel (II)

In einer Rührapparatur wurden 450 ml konz. Schwefelsäure vorgelegt und bei 45 bis 50 °C 195 g eines Dichlor- oder Trichlor-trifluormethyl-benzalchlorids zugetropft. Es wurde 4 Stunden nachgerührt und dann auf Eis gegossen. Die organische Phase wurde abgetrennt und im Vakuum destilliert. Es fielen zwischen 100 und 138 g des entsprechenden Dichlor- oder Trichlor-trifluormethylbenzaldehyds an.

Auf diese Weise wurden erhalten:

a) 2,6-Dichlor-3-trifluormethylbenzaldehyd (Sdp: 125-128 °C/24 mbar, Fp: 48-50 °C)
b) 4,5-Dichlor-2-trifluormethylbenzaldehyd (Sdp: 106 °C/20 mbar, $n_D^{20}$ : 1,5165)
c) 2,3-Dichlor-4-trifluormethylbenzaldehyd (Sdp: 114-117 °C/20 mbar),
d) 2,4-Dichlor-5-trifluormethylbenzaldehyd (Sdp: 105-107 °C/14 mbar),
e) 2,6-Dichlor-4-trifluormethylbenzaldehyd (Sdp: 110-113 °C/15 mbar),
f) 2,3,4-Trichlor-5-trifluormethylbenzaldehyd,
g) 2,4,6-Trichlor-5-trifluormethylbenzaldehyd,
h) 2,3,6-Trichlor-4-trifluormethylbenzaldehyd,
i) 2,3,6-Trichlor-5-trifluormethylbenzaldehyd,
j) 2,3,4-Trichlor-6-trifluormethylbenzaldehyd,
k) 3,4,5-Trichlor-2-trifluormethylbenzaldehyd,
l) 2,5-Dichlor-4-trifluormethylbenzaldehyd und
m) 2,3,5-Trichlor-4-trifluormethylbenzaldehyd.

Beispiel 6

2-Fluor-3-trifluormethyl-benzaldehyd

109 g Kaliumfluorid wurden zusammen mit 210 ml Tetramethylensulfon vorgelegt und im Vakuum zur Trocknung andestilliert. Dann wurden 10 g 18-Krone-6 und 218 g 2-Chlor-3-trifluormethyl-benzaldehyd zugegeben und unter Feuchtigkeitsanschluß für 5 Stunden auf 190 °C erhitzt (GC; quantitativer Umsatz). Die Destillation im Vakuum lieferte 157 g 2-Fluor-3-trifluormethyl-benzaldehyd (Siedepunkt 60 °C/20 mbar).

## Patentansprüche

**1.** Verfahren zur Herstellung von kernfluorierten Trifluormethylbenzaldehyden der Formel (I)

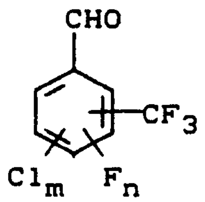

(I),

in der
n für 1, 2 oder 3 und
m für 0, 1 oder 2 steht, wobei
n + m maximal 3 beträgt,
dadurch gekennzeichnet, daß man einen kernchlorierten Trifluormethylbenzaldehyd der Formel (II),

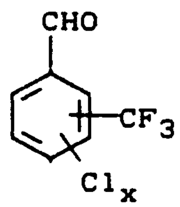

(II),

in der
x für 1, 2 oder 3 steht,
mit Kaliumfluorid bei erhöhter Temperatur in Gegenwart eines Lösungsmittels und in Abwesenheit von Wasser umsetzt.

**2.** Verfahren nach Anspruch 1, dadurch gekennzeichnet, daß man 1 bis 2 Mol Kaliumfluorid pro Mol gegen Fluor auszutauschendes Chlor einsetzt.

**3.** Verfahren nach Ansprüchen 1 und 2, dadurch gekennzeichnet, daß man es bei Temperaturen im Bereich 160 bis 210 °C durchführt.

**4.** Verfahren nach Ansprüchen 1 bis 3, dadurch gekennzeichnet, daß man ein polares, aprotisches Lösungsmittel verwendet.

**5.** Verfahren nach Ansprüchen 1 bis 4, dadurch gekennzeichnet, daß man es in Gegenwart von Katalysatoren durchführt.

**6.** Kernfluorierte Trifluormethylbenzaldehyde der Formel (Ia)

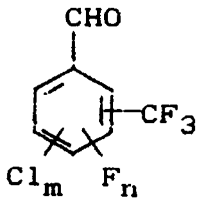

(Ia),

in der

n für 1, 2 oder 3 steht und

m für 0, 1 oder 2 steht, wobei

n + m 2 oder 3 beträgt und

wobei die gemäß EP-A 125 803 und EP-A 174 131 hergestellten kernfluorierten Trifluormethylbenzaldehyde ausgenommen sind.

**7.** Kernfluorierte Trifluormethylbenzaldehyde gemäß Anspruch 6, dadurch gekennzeichnet, daß es sich um
2-Trifluormethyl-4,6-difluor-benzaldehyd,
2-Trifluormethyl-4-fluor-6-chlor-benzaldehyd,
2-Trifluormethyl-4-fluor-5-chlor-benzaldehyd,
2-Trifluormethyl-4,5-difluor-benzaldehyd,
3-Trifluormethyl-4,5-difluor-benzaldehyd,
3-Trifluormethyl-4-fluor-5-chlor-benzaldehyd,
2,4-Difluor-3-trifluormethyl-benzaldehyd,
3-Trifluormethyl-4,6-difluor-benzaldehyd,
2-Fluor-4-trifluormethyl-5-chlor-benzaldehyd,
2-Fluor-3-chlor-4-trifluormethyl-benzaldehyd,
2,4-Difluor-4-trifluormethyl-benzaldehyd,
2,6-Difluor-4-trifluormethyl-benzaldehyd,
2-Chlor-4-trifluormethyl-6-fluor-benzaldehyd,
3-Chlor-4-trifluormethyl-6-fluor-benzaldehyd,
2,6-Difluor-4-trifluormethyl-5-chlor-benzaldehyd,
2,5,6-Trifluor-3-trifluormethyl-benzaldehyd,
2,6-Difluor-3-trifluormethyl-5-chlor-benzaldehyd,
2,4,6-Trifluor-3-trifluormethyl-benzaldehyd,
2-Trifluormethyl-3-chlor-4,6-difluor-benzaldehyd
oder 2-Trifluormethyl-3,4,6-trifluor-benzaldehyd handelt.

## Claims

**1.** Process for the preparation of nuclear-fluorinated trifluoromethylbenzaldehydes of the formula (I)

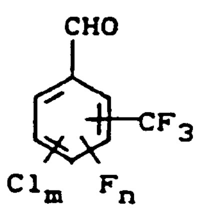

(I)

in which

n represents 1, 2 or 3 and

m represents 0, 1 or 2,

n + m being not more than 3,

characterized in that a nuclear-chlorinated trifluoromethylbenzaldehyde of the formula (II)

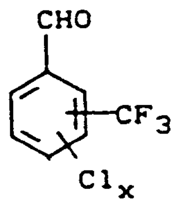

(II)

in which

x represents 1, 2 or 3,

is reacted with potassium fluoride at an elevated temperature in the presence of a solvent and in the absence of water.

2. Process according to Claim 1, characterized in that 1 to 2 moles of potassium fluoride are employed per mole of chlorine to be replaced by fluorine.

3. Process according to Claims 1 and 2, characterized in that it is carried out at temperatures within the range from 160 to 210 °C.

4. Process according to Claims 1 to 3, characterized in that a polar, aprotic solvent is used.

5. Process according to Claims 1 to 4, characterized in that it is carried out in the presence of catalysts.

6. Nuclear-fluorinated trifluoromethylbenzaldehydes of the formula (Ia)

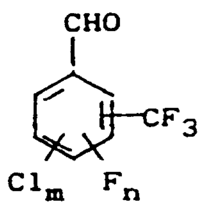

(Ia)

in which

n represents 1, 2 or 3 and

m represents 0, 1 or 2,

n + m being 2 or 3 and

the nuclear-fluorinated trifluoromethylbenzaldehydes prepared in accordance with EP-A 125,803 and EP-A 174,131 being excepted.

7. Nuclear-fluorinated trifluoromethylbenzaldehydes according to Claim 6, characterized in that they are 2-trifluoromethyl-4,6-difluorobenzaldehyde, 2-triflouromethyl-4-fluoro-6-chlorobenzaldehyde, 2-trifluoromethyl-4-fluoro-5-chlorobenzaldehyde, 2-trifluoromethyl-4,5-difluorobenzaldehyde, 3-trifluoromethyl-4,5-difluorobenzaldehyde, 3-trifluoromethyl4-fluoro-5-chlorobenzaldehyde, 2,4-difluoro-3-trifluoromethylbenzaldehyde, 3-trifluoromethyl-4,6-difluorobenzaldehyde, 2-fluoro-4-trifluoromethyl-5-chlorobenzaldehyde, 2-fluoro-3-chloro-4-trifluoromethylbenzaldehyde, 2,4-difluoro-4-trifluoromethylbenzaldehyde, 2,6-difluoro-4-trifluoromethylbenzaldehyde, 2-chloro-4-trifluoromethyl-6-fluorobenzaldehyde, 3-chloro-4-trifluoromethyl-6-fluorobenzaldehyde, 2,6-difluoro-4-trifluoromethyl-5-chlorobenzaldehyde, 2,5,6-trifluoro-3-trifluoromethylbenzaldehyde, 2,6-difluoro-3-trifluoromethyl5-chlorobenzaldehyde, 2,4,6-trifluoro-3-trifluoromethylbenzaldehyde, 2-trifluoromethyl-3-chloro-4,6-difluorobenzaldehyde or 2-trifluoromethyl-3,4,6-trifluorobenzaldehyde.

## Revendications

1. Procédé de préparation de trifluorométhylbenzaldéhydes à noyau fluoré de formule (I)

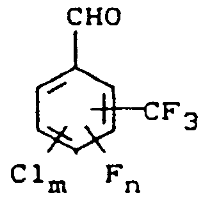

(I),

dans laquelle
n représente 1, 2 ou 3 et
m représente 0, 1 ou 2,
n + m s'élevant au maximum à 3,
caractérisé en ce qu'on fait réagir un trifluorométhylbenzaldéhyde à noyau chloré de formule (II),

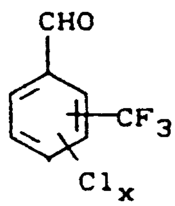

(II),

dans laquelle
x représente 1, 2 ou 3,
avec du fluorure de potassium à température élevée en présence d'un solvant et en l'absence d'eau.

2. Procédé selon la revendication 1, caractérisé en ce qu'on introduit de 1 à 2 mol de fluorure de potassium par mole de chlore à échanger contre du fluor.

3. Procédé selon la revendication 1 ou 2, caractérisé en ce qu'il est mis en oeuvre à des températures situées dans un intervalle de 160 à 210 °C.

4. Procédé selon les revendications 1 à 3, caractérisé en ce qu'on utilise un solvant aprotique polaire.

5. Procédé selon les revendications 1 à 4, caractérisé en ce qu'il est mis en oeuvre en présence de catalyseurs.

6. Trifluorométhylbenzaldéhydes à noyau fluoré de formule (Ia)

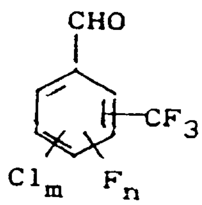

(Ia),

dans laquelle
n représente 1, 2 ou 3 et
m représente 0, 1 ou 2,
n + m valant 2 ou 3 et
d'où les trifluorométhylbenzaldéhydes à noyau fluoré préparés selon les documents EP-A 125 803 et

EP-A 174 131 sont exclus.

7. Trifluorométhylbenzaldéhydes à noyau fluoré selon la revendication 6, caractérisé en ce qu'il s'agit de :
2-trifluorométhyl-4,6-difluorobenzaldéhyde,
2-trifluorométhyl-4-fluoro-6-chlorobenzaldéhyde,
2-trifluorométhyl-4-fluoro-5-chlorobenzaldéhyde,
2-trifluorométhyl-4,5-difluorobenzaldéhyde,
3-trifluorométhyl-4,5-difluorobenzaldéhyde,
3-trifluorométhyl-4-fluoro-5-chlorobenzaldéhyde,
2,4-difluoro-3-trifluorométhylbenzaldéhyde,
3-trifluorométhyl-4,6-difluorobenzaldéhyde,
2-fluoro-4-trifluorométhyl-5-chlorobenzaldéhyde,
2-fluoro-3-chloro-4-trifluorométhylbenzaldéhyde,
2,4-difluoro-4-trifluorométhylbenzaldéhyde,
2,6-difluoro-4-trifluorométhylbenzaldéhyde,
2-chloro-4-trifluorométhyl-6-fluorobenzaldéhyde,
3-chloro-4-trifluorométhyl-6-fluorobenzaldéhyde,
2,6-difluoro-4-trifluorométhyl-5-chlorobenzaldéhyde,
2,5,6-trifluoro-3-trifluorométhylbenzaldéhyde,
2,6-difluoro-3-trifluorométhyl-5-chlorobenzaldéhyde,
2,4,6-trifluoro-3-trifluorométhylbenzaldéhyde,
2-trifluorométhyl-3-chloro-4,6-difluorobenzaldéhyde
ou 2-trifluorométhyl-3,4,6-trifluorobenzaldéhyde.